# DEMANDE DE BREVET EUROPEEN

(11) **EP 0 118 347 A1**
(43) Date de publication de la demande: **12.09.1984**
(21) Numéro de dépôt: 84400313.7
(22) Date de dépôt: 15.02.1984
(51) Int. Cl.: C07C 103/78, A61K 49/04

(54) **Composés non-ioniques à structure benzénique iodée ou bromée et produits opacifiants en contenant**

(30) Priorité: 25.02.1983 FR 8303141
(71) Demandeur: GUERBET S.A., F-93601 Aulnay-sous-Bois Cedex (FR)
(72) Inventeur: Dimo, Ioana, F-94160 Saint Mande (FR); Bonnemain, Bruno, F-77290 Mitry Mory (FR); Hardouin, Michel Jean-Charles, F-94120 Fontenay sous Bois (FR); Lautrou, Jean, F-94160 Saint Mande (FR)
(74) Mandataire: Bressand, Georges

(57) **Abrégé**

La présente invention a pour objet des composés de formule I 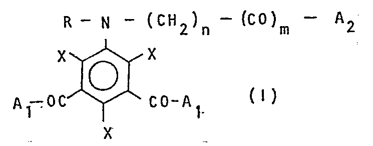 dans laquelle
X est un atome d'iode ou de brome,
R est un atome d'hydrogène, un groupe acétyle, méthoxyacétyle ou trifluoroacétyle,
m est un nombre entier de 0 à 2,
n est un nombre entier de 0 à 4,
la somme m + n étant au moins égale à 2,
A₁ et A₂ représentent indépendamment l'un de l'autre un reste d'aminoalcool

Ces composés sont utilisables dans des produits opacifiants.

## Description

La présente invention concerne des composés utilisables dans des produits opacifiants pour la radiographie.

On utilise depuis longtemps comme produits opacifiants des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suf₋ fisante en vue de l'administration de ces composés en solution aqueuse et, d'autre part,.pour conférer à ces composés une tolérance suffisante par l'organisme humain.

A cet effet, on a proposé des structures non-ioniques, c'est-à-dire des dérivés iodobenzéniques possédant des substituants non-ioniques.

Ainsi, dans le brevet FR-A-2 053 037, on a proposé des composés carbamoyl iodobenzéniques contenant au total au moins un groupe N-hydroxy alcoyle et au moins deux groupes hydroxy.

Un composé illustrant cette classe est le métri- zamide qui s'avère être toutefois d'une stabilité limitée.

Plus récemment, on a proposé d'autres composés iodobenzéniques non-ioniques à groupes N-hydroxyalcoyle (Brevets FR-A-2 293 919 et FR-A-2 354 316).

La présenté invention vise à fournir de nouveaux composés à groupes N-hydroxyalcoyle qui soient bien tolérés par l'organisme humain, très stables en solution aqueuse et qui puissent être obtenus aisément et avec un faible prix de revient.

La présente invention a ainsi pour objet des composés de formule 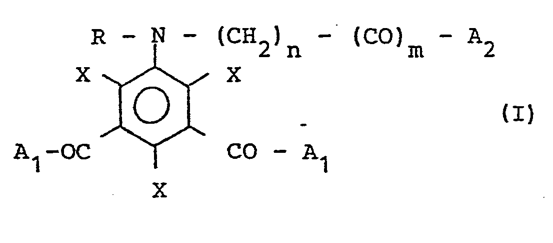 dans laquelle
X est un atome d'iode ou de brome,
R est un atome d'hydrogène, un groupe acétyle, méthoxyacétyle ou trifluoroacétyle,
m est un nombre entier de 0 à 2,
n est un nombre entier de 0 à 4,
   la somme m + n étant au moins égale à 2,
A₁ et A₂ représentent indépendamment l'un de l'autre un reste d'aminoalcool de formule 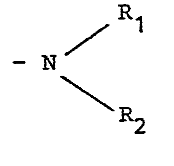
dans laquelle
R₁ représente un atome d'hydrogène, un groupe alcoyle en C₁ à C₆ ou un groupe alcoyle en C₁ à C₆ mono-ou polyhydroxylé, et
R₂ représente un groupe alcoyle en C₁ à C₆ mono-ou polyhydroxylé.

Un groupe avantageux de composés de formule I est constitué par les composés de formule 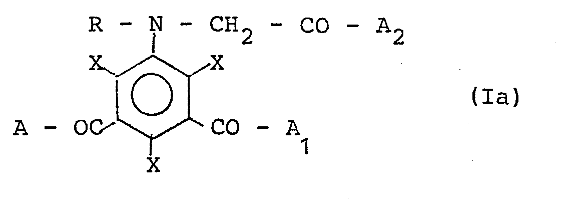 dans laquelle X, R, A₁ et A₂ ont la signification donnée ci-dessus.

Un autre groupe avantageux de composés de formule I est constitué par les composés de formule 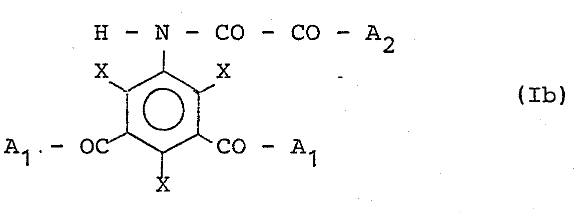 dans laquelle X, A₁ et A₂ ont la signification donnée ci-dessus.

Parmi les composés de formule I, Ia ou Ib, on préfère ceux dans lesquels les restes d'aminoalcool A₁ et A₂ comprennent au moins deux groupes hydroxy.

Les composés de formule I peuvent être préparés aisément à partir des composés de formule 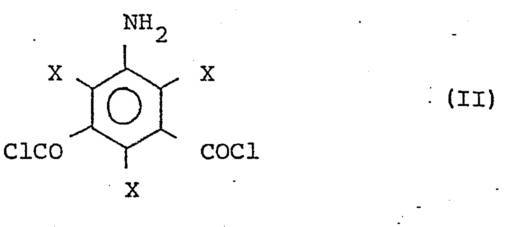 dans laquelle X a la signification donnée ci-dessus, par condensation avec une amine de formule 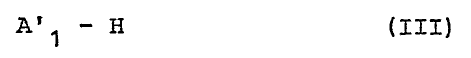 dans laquelle A'₁ désigne un groupe A₁ dont les groupes hydroxy sont protégés conduisant à une amine de formule 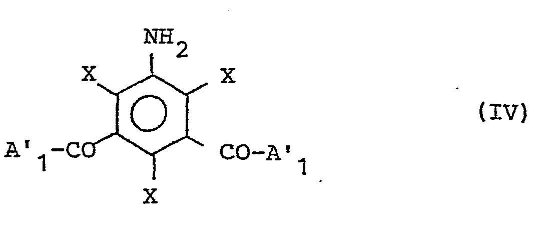 éventuellement acylation de cette amine par un chlorure d'acide de formule R' Cl (V) dans laquelle R' représente un groupe R de type acyle, obtenant ainsi un composé cie formule 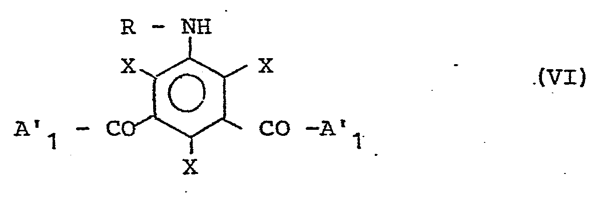 puis réaction de ce composé de formule (VI) avec soit un composé de formule 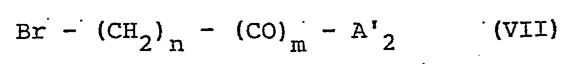 dans laquelle A'₂ est un groupe A₂ dont les groupes hydroxy sont protégés et n > 0, soit un composé de formule 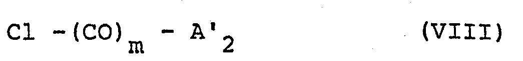 dans laquelle A'₂ est un groupe A₂ dont les groupes hydroxy sont protégés conduisant à un composé de formule 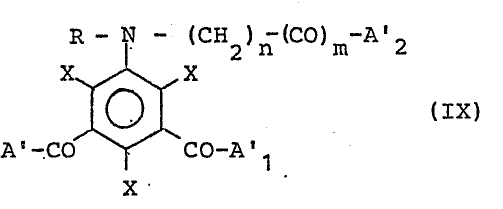 et élimination des groupes protecteurs.

En variante, on peut également inverser l'ordre des réactions, c'est-à-dire effectuer d'abord une acylation de l'amine de formule II avec un chlorure d'acide de formule (V), conduisant à un dichlorure d'acide de formule 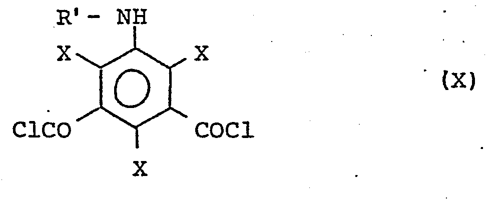 puis une condensation de ce chlorure d'acide (X) avec une amine de formule (III), conduisant ainsi à un composé de formule (VI).

On peut également, en variante, utiliser au lieu d'une amine de formule (III) une amine non protégée de formule A₁H (XI) et n'effectuer la protection des groupes hydroxy qu'ultérieurement.

On peut en outre utiliser comme produit de départ un diester de l'acide isophtalique de formule 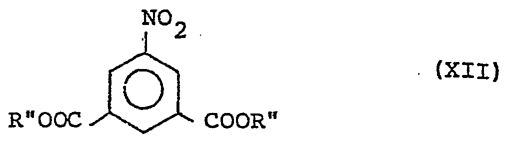 R" désignant un groupe alcoyle en C₁ à C₄ et condenser ce diester avec une amine de formule XI conduisant à un composé de formule 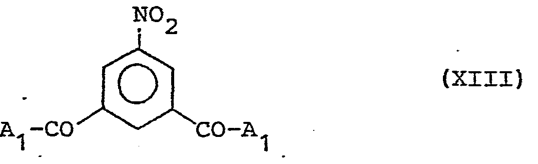 puis effectuer successivement une réduction du groupe nitro de ce composé, puis une halogénation du noyau benzénique, obtenant ainsi un composé de formule 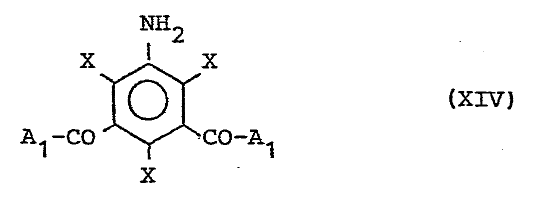 que l'on peut ensuite protéger pour obtenir un composé de formule VI.

Les composés iodobenzéniques de formule (I) peuvent être utilisés en radiographie classique, notamment en angiographie et en myélographie.

Les composés bromobenzéniques de formule (I) peuvent être utilisés notamment en radiographie numérisée, en particulier en angiographie numérisée.

De plus, les composés bromobenzéniques de formule (I) peuvent être utilisés en mélange avec des composés iodobenzéniques qui peuvent être soit des composés iodobenzéniques de formule (I), soit d'autres composés iodobenzéniques. Ces autres composés peuvent être notamment des composés non-ioniques, tels que le 5-(N-2,3-dihydroxy- propyl-N-acétyl-amino)-2,4,6-triiodo-N,N'-bis (2,3-dihy- droxypropyl) isophtalamide et le 5-(N-2-hydroxypropionyl- amino)-2,4,6-triiodo-N,N'-bis (1,3-dihydroxy-2-propyl) isophtalamide ou des composés ioniques tels que l'acide diatrizoique, l'acide iothalamique, l'acide métrizoïque, l'acide acétrizoique, l'iodamide, l'acide ioxitalamique, le ioglunide, l'acide ioxaglique, l'acide iocarmique, l'adipiodone et l'acide ioglycamique.

La Demanderesse a en effet constaté qu'en utilisant des mélanges de tels composés on obtient une tolérance plus grande qu'avec les seuls composés iodobenzéniques, tout en ayant une opacité du même ordre de grandeur. Ceci est d'autant plus intéressant que le brome est actuellement bien moins onéreux que l'iode.

Dans ces mélanges, les composés bromobenzéniques de formule (I) représentent avantageusement de 1/2 à 2/1 (en moles) des composés iodobenzéniques.

La présente invention a donc également pour objet des produits opacifiants qui comprennent des composés iodobenzéniques de formule (I), des composés bromobenzéniques de formule (I), des mélanges de composés bromobenzéniques de formule (I) avec des composés iodobenzéniques de formule (I) ou des mélanges de composés bromobenzéniques de formule (I) avec d'autres composés iodobenzéniques.

La forme pharmaceutique préférée des produits opacifiants selon l'invention est constituée par des solutions aqueuses de ces composés.

Les solutions aqueuses contiennent généralement un total de 5 à 100 g de composés iodobenzéniques et/ou bromobenzéniques pour 100 ml et la quantité injectable de telles solutions peut varier généralement de 5 à 1000 ml.

On donnera ci-après des exemples de compositions selon la présente invention.

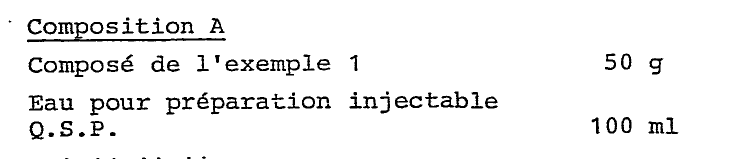

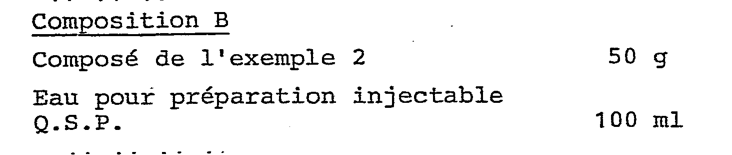

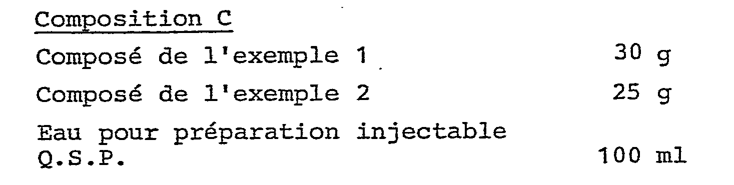

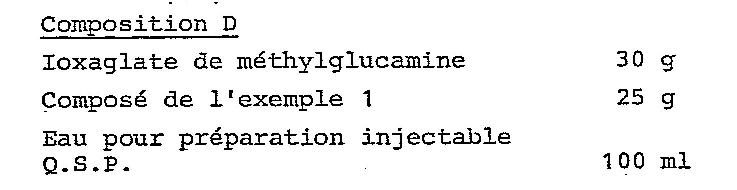

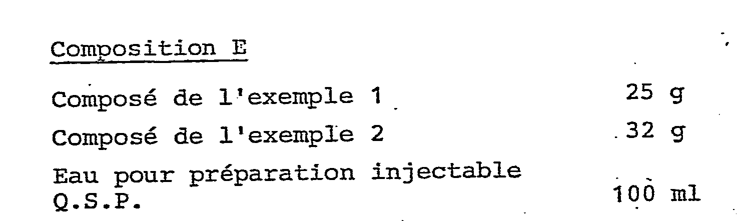

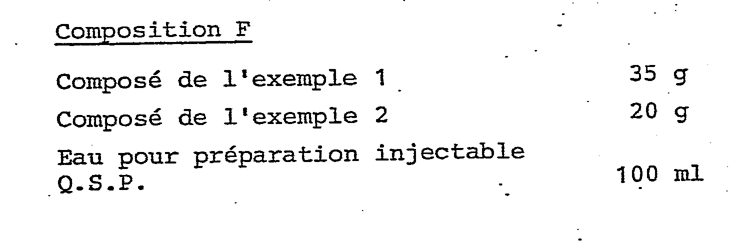

Les exemples suivants illustrent la présente invention.

Dans ces exemples, les composés ont été caractérisés par chromatographie en couche mince mesurée sur plaque de silicagel F254 Merck avec les éluants suivants:

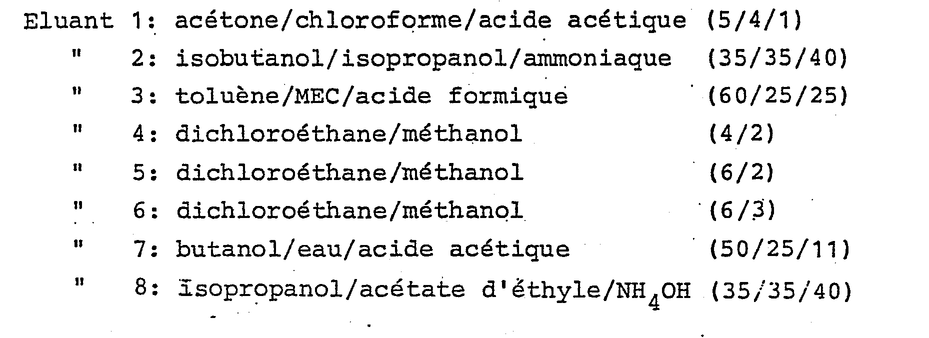

### EXEMPLE 1

### Préparation du 5-[N-bis (β-hydroxyéthyl) carbamoylméthyl-N -acétyl-amino]-2,4,6-triiodo-N,N'-bis [bis (S-hydroxyéthyl]% isophtalamide

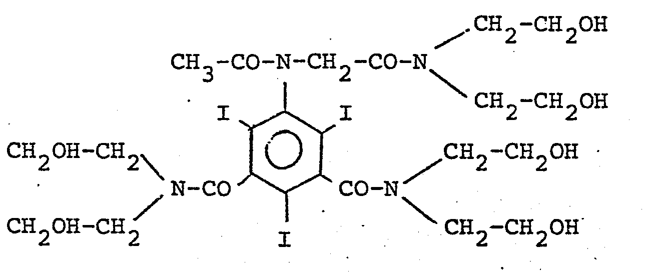

### a) Préparation du chlorure de l'acide 5-amino-2,4,6-triiodo isophtalique

1,5 kg de l'acide 5-amino-2,4,6-triiodo isophtalique est mis en suspension avec 4,025 litres de chlorure de thionyle et 5 ml de diméthylformamide. Le mélange réactionnel est chauffé 5 heures à 60°C. Après évaporation du chlorure de thionyle en excès sous vide, le produit est lavé avec 4 litres d'eau, puis filtré et claircé à l'éther diisopropylique.

Le produit obtenu est ensuite séché à l'air, puis sous vide. Rendement: 87,3 %.
CCM : Eluant 1 Rf: 1 (solution à 2 % dans l'acétone)

Les spectres IR et ¹HRMN confirment la structure.

### b) Préparation du 5-amino-2,4,6-triiodo-N,N'-bis [bis (β-hydroxyéthyl)] isophtalamide

1,6 kg (2,68 moles) du chlorure de l'acide 5-amino-2,4,6-triiodo isophtalique est dissous dans 4 litres de diméthylacétamide en présence de 1,13 1 de tri- éthylamïne (8,04 moles).

La diéthanolamine (775 ml, 8,04 moles) est ajoutée progressivement en maintenant la température du milieu réactionnel entre 15 et 20°C. La réaction est laissée sous agitation une nuit à température ambiante. Après essorage du chlorhydrate de triéthylamine, le diméthylacétamide est évaporé à sec.

L'huile résultante est dissoute dans l'eau et cristallise après une nuit à température ambiante.

Le produit est filtré, puis séché sous vide. Rendement: 73,8 %.

CCM: Eluant 2 - Rf : 0,67, 0,63 (présence de 2 atropisomères).

### c) Préparation du 5-acétamido-2,4,6-tiiodo-N,N'-bis /bis (β-acétoxyéthyl)] isophtalamide

1,45 kg (1,98 mole) de 5-amino-2,4,6-triiodo-N,N'- bis [bis (β-hydroxyéthyl)]isophtalamide est dissous dans 2,2 1 d'éther diméthylique de l'éthylène glycol. Après addition de 1,06 litre (14,85 moles) de chlorure d'acétyle, la solution est chauffée à 80°C. La cristallisation s'effectue spontanément après une nuit à température ambiante.

Après filtration et séchage, on récupère 1,58 kg de produit. Rendement: 85 %.
CCM : Eluant 3 Rf : 0,25.

### d) Préparation du N-bis (β-acétoxyéthyl)-α-bromo acétamide

### a) N-bis (β-acétoxyéthyl) amine, chlorhydrate

15 g de diéthanolamine sont ajoutés progressivement à une solution de 0,6 1 d'acide acétique saturé à 20°_{C} en acide chlorhydrique gazeux. Après une nuit, la solution est évaporée à sec, puis reprise par l'éther diisopropylique, .le produit est filtré, puis séché sous vide. Rendement: 96 %.

### β) N-bis (β-acétoxyéthyl)-α-bromo-acétamide

16,9 g (0,075 mole) de N-bis (β-acétoxyéthyl) amine sont dissous dans 65 ml d'une solution de dichlo- réthane contenant 6,85 g d'hydrogénocarbonate de sodium. Cette solution est chauffée 1 heure à 50°C. Après essorage du chlorure de sodium formé, séchage sur sulfate de sodium, puis filtration, on ajoute entre 10 et 15°C 13,7 g de bromure de bromacétyle (0,068 mole), puis 6,9 g (0,068 mole) de triéthylamine. La réaction est laissée une nuit à température ambiante, le bromhydrate de triéthylamine est essoré. Les eaux mères récupérées sont lavées à l'eau, séchées sur sulfate de sodium, puis évaporées à siccité.

On récupère 16,3 g d'une huile. Rendement: 77 %.
CCM: acétate d'éthyle/chlorure de méthylène (25/25) Rf: 0,63.

Les spectres IR et ¹HRMN sont en accord avec la structure.

### e) Préparation du 5-/N-bis (S-hydroxyéthyl) carbamoyl méthyl-N-acétyl-amine]-2,4.6-triiodo-N,N'-bis [bis-(β-hydroxyéthyl)] isophtalamide

200 g de 5-acétamido-2,4,6-triiodo-N,N'-bis /bis (β-acétoxyéthyl)] isophtalamide sont ajoutés à une suspension de 5,6 g (0,234 mole) d'hydrure de sodium dans 340 ml de diméthylformamide anhydre. Après 1 heure de réaction 72,3 g (0,233 mole) de N-bis (acétoxyéthyl)-a-bromoacétamide sont additionnés à température ambiante. Le mélange réactionnel est laissé une nuit à température ambiante, puis évaporé à sec.

L'huile résultante est saponifiée par dissolution dans 683 ml de soude 2N (1,366 mole) et 300 ml d'éthanol

Après 1 heure d'agitation, on ajoute 3,4 1 d'eau, puis 1,08 1 de résine cationique Amberlite IRN77. Après filtration, les eaux mères sont traitées par 1,46 1 de résine anionique IRN78. La suspension est filtrée et les jus sont évaporés à sec.

Le produit blanc.cristallin récupéré est recristallisé dans 700 ml de butanol. Rendement: 73 %.
CCM: Eluant 4 - Rf: 0,2 et 0,25 (atropisomères)..

L'analyse ¹HRMN et IR confirme la structure.

### EXEMPLE 2

### Préparation du 5-[N-bis (β-hydroxyéthyl) carbamoylméthyl-N-acétyl-amino]-2,4,6-tribromo-N,N'-bis-[bis (β-hydroxyéthyl)] isophtalamide

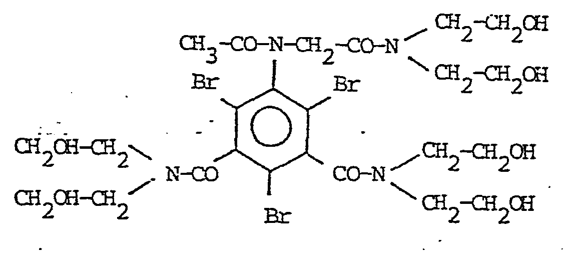

### a) Préparation du chlorure de l'acide 5-amino-2,4,6-tribromo isophtaligue

500 g de l'acide 5-amino-2,4,6-tribromo isophtalique (1,2 mole) sont mis en suspension avec 3 1 de chlorure de thionyle et 1 ml de diméthyl formamide. Le mélange est porté au reflux pendant 4 heures.

Après évaporation du chlorure de thionyle en excès, le produit est lavé dans 2 1 d'éther diisopropylique. La totalité du produit est précipitée par addition d'éther de pétrole. Le dichlorure est filtré, puis claircé à l'éther de pétrole et séché sous vide. Rendement : 90 %. CCM : Eluant 7. Rf : 0,9.

### b) Préparation du 5-amino- 2,4,6-tribromo-N,N'-bis [bis (β-hydroxyéthyl)] isophtalamide

363 g de chlorure de l'acide 5-amino-2,4,6-tribromo- isophtalique sont dissous dans 380 ml d'acétone. La diéthanolamine (236 ml) en solution dans 200 ml d'eau et 236 g d'hydrogénocarbonate de potassium est ajoutée lentement en maintenant la température du milieu réactionnel aux environs de 20°C.

Après plusieurs jours d'agitation à température ambiante le produit cristallise.

Après filtration et séchage sous vide, le produit est récristallisé dans 1500 ml d'éthanol absolu. Rendement : 70 %.
CCM : Eluant 7 Rf : 0,55 et 0,65.
Eluant 6 Rf : 0,48 et 0,52.

### c) Préparation du 5-acétamido-2,4,6-tribromo-N,N'-bis /bis (β-acétoxyéthyl)] isophtalamide

10 g (0,016 mole) du 5₋amino-2,4,6-tribromo-N, N'-bis /bis (β-hydroxyéthyl)] isophtalamide sont dissous dans 20 ml de l'éther diméthylique de l'éthylène glycol. Après l'addition de 9,52 ml de chlorure d'acétyle, la solution est chauffée à 80°C pendant 45 minutes, la solution est ensuite refroidie à température ambiante et laissée une nuit sous agitation. Le produit cristallisé blanc est filtré, lavé à l'éther diéthylique, puis séché à l'étuve. Rendement : 74 %.
CCM : Eluant 7 Rf : 0,72

Eluant 6 Rf : 0,88.

### d) Préparation du 5-/N-bis (β-hydroxyéthyl) carbamoylméthyl-N-acétyl-amino7-2,4,6-tribromo-N,N'-bis [bis (β-hydroxyéthyl)] isophtalamide

5 g (6,2 moles) de 5-acétamido-2,4,6-tribromo-N,N'-bis /bis (β-acétoxyéthyl)] isophtalamide sont ajoutés à une suspension de 0,299 g de NaH (7,5 mmoles) dans 10 ml de diméthylacétamide anhydre. Après une heure de réaction(dégagement d'hydrogène), 2,32 g de N-bis (β-acétoxy éthyl)-α-bromoacétamide dilués dans 2 ml de DMAC sont additionnés. Le milieu réactionnel est laissé une nuit à température ambiante, puis évaporé à sec.

L'huile résultante est saponifiée par dissolution dans 8 ml d'éthanol et 20 ml de soude 2N. La solution est ensuite traitée successivement par une résine cationique (Amberlite IRN77) et par une résine anionique (Amberlite IRN78). Après filtration, les jus sont évaporés à sec.

Le produit résultant est recristallisé dans le butanol. Le rendement est de 70 %.
CCM : Eluant 7 Rf : 0,35 et 0,40
Eluant 5 (60/40) Rf : 0,22 et 0,28.

Dosage du brome : 98 %.

### EXEMPLE 3

### Préparation du 5-/N-tris (hydroxyméthyl) méthyl-carbamoylméthyl-N-acétyl-amino]-2,4,6-triiodo-N,N-bis Ibis (β-hydroxyéthyl)] isophtalamide

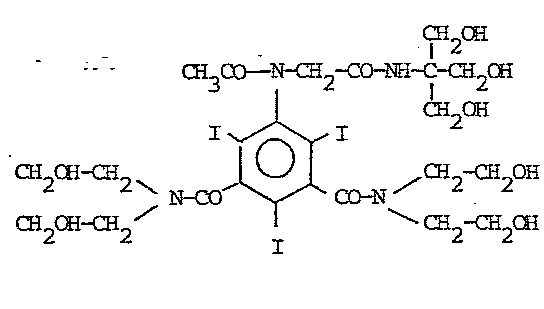

### a) Préparation du tris (acétoxyinéthyl) aminonséthane chlorhydrate

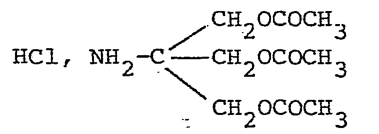

-100 g du chlorhydrate de tris (hydroxyméthyl) aminométhane sont dissous à 100°C dans 240 g d'anhydride acétique et 320 g d'acide acétique. Le mélange réaction- neo est chauffé à 100°C pendant 10 heures, puis évaporé à sec. L'huile résiduelle est lavée plusieurs fois à l'éther, puis évaporée àsec. 144.g d'une huile incolore sont récupérés (73 % de rendement).

L'analyse spectrale HRMN et IR confirme la structure.

### b) Préparation du N-(tris-acétoxyméthyl) méthyl-a-bromo acétamide

Le composé obtenu en (a) est traité comme décrit à l'exemple 1 ((d) β) .

On obtient le N-(tris-acétoxyméthyl) méthyl-a-bromoacétamide avec un rendement de 75 %.

L'analyse ¹HRMN et IR confirme la structure.

### c) Préparation du 5-/N-tris (hydroxyméthyl) méthyl-carbamoylméthyl-N-acétyl-amino]-2,4,6-triiodo-N,N'-bis- /bis (β-hydroxyéthyl)] isophtalamide

On opère comme décrit à l'exemple 1(e) en utilisant le composé obtenu en (b) à la place du N-bis (acétoxyéthyl)-a-bromo-acétamide.

### EXEMPLE 4

### Préparation du 5-/N-tris (hydroxyméthyl) méthyl-carbamoylméthyl-N-acétyl-amino]-2,4,6-tribromo-N,N'-bis [bis(β-hydroxyéthyl] isophtalamide

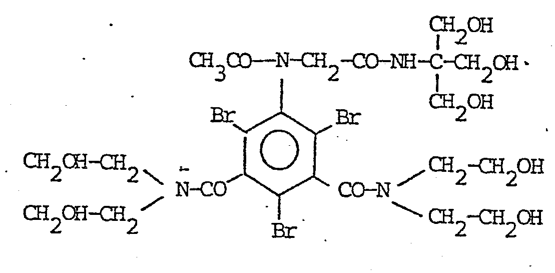

Il est obtenu comme décrit à l'exemple 2(d) en utilisant le composé obtenu à l'exemple 3(b) à la place du N-bis (acétoxyéthyl)-α-bromoacétamide.

### EXEMPLE 5

### Préparation du 5-[N-bis (β-hydroxyéthyl) carbamoyl-méthyl-N-trifluoroacétyl-amino]-2,4,6-tribromo-N,N'-bis /bis (β-hydroxyéthyl]lisophtalamide

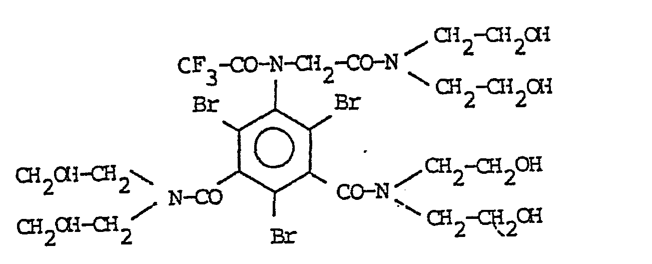

### a) Préparation du 5-trifluoroacétamido-2,4,6-tribromo-N,N'-bis [bis (β-trifluoro-acétoxyéthyl)] isophtalamide

10 g (0,016 mole) du 5-amino-2,4,6-tribromo-N, N'-bis [bis (β-hydroxyéthyl)] isophtalamide dissous dans 20 ml de l'éther diméthylique de l'éthylène glycol sont trifluoroacétylés en présence de 33,6 g (0,16 mole) d'anhydride trifluoroacétique à 80°C pendant 4 heures. Après une nuit à -10°C, on récupère un précipité blanc qui est lavé à l'éther isopropylique, puis séché à l'étuve.

### b) Préparation du 5-[N-bis (S-hydroxyéthyl) carbamoylméthyl-N-trifluoroacétyl-amino/-2,4,6-tribromo-N,N'- bis /bis (β-hydroxyéthyl)]isophtalamide

Il est obtenu à partir du composé obtenu en (a) comme décrit à l'exemple 1(e).

### EXEMPLE 6

### Préparation du 5-[N-bis (β-hydroxyéthyl) carbamoylméthyl-N-trifluoro-acétyl-amino]-2,4,6-triiodo-N,N'-bis [bis (β-hydroxyéthyl)] isophtalamide

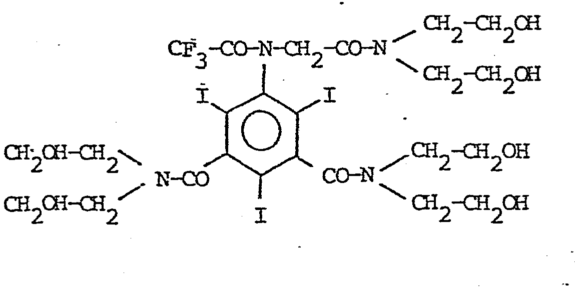

Il est obtenu comme décrit à l'exemple 5 en partant du 5-amino-2,4,6-triiodo-N,N'-bis /bis (S-hydroxyéthyl)] isophtalamide.

### EXEMPLE 7

### Préparation du 5-[N-(2,3-dihydroxypropyl-carbamoyl-méthyl)-N-acétyl-amino]-2,4,6-triiodo-N,N'-bis (2,3-dihydroxypro- pyl) isophtalamide

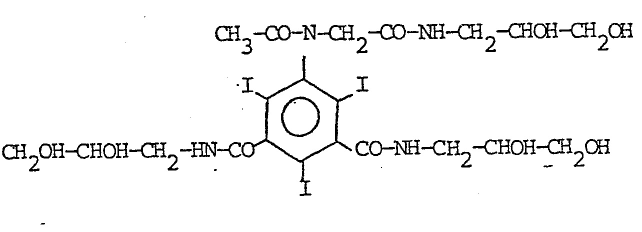

Il est obtenu comme décrit à l'exemple 1.

### EXEMPLE 8

### Préparation du 5-[N-(2,3-dihydroxypropyl-carbamoyl-méthyl)-N-acétylamino7-2,4,6-tribromo-N,N'-bis (2,3-dihydroxypro- pyl) isophtalamide

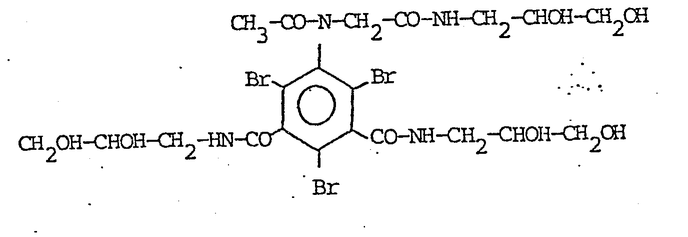

Il est obtenu comme décrit à l'exemple 2.

### EXEMPLE 9

### Préparation du 5-/N-bis (β-hydroxyéthyl) carbamoyl car- bonyl-amino]-2,4,6-triiodo-N,N'-bis /bis (β-hydroxyethyl)] isophtalamide

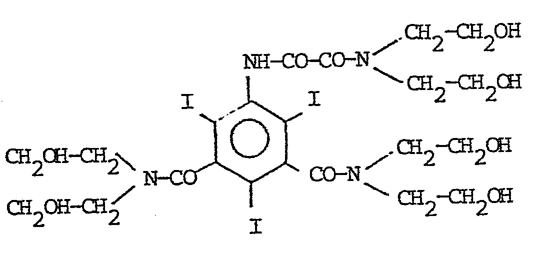

### a) Préparation du sel de potassium de l'acide N-bis (β-hvdroxyéthyl) oxamique

On dissout 156 g (1 mole) d'éthoxalate de potassium dans 300 ml d'eau, on ajoute 150 g (1,4 mole) de diéthanolamine. On agite pendant une nuit à température ambiante, puis pendant 6 heures à 50°C et on laisse recristalliser pendant 24 heures. Après essorage, lavage à l'éthanol absolu, on obtient un premier jet de 35 g. Après évapôra- tion des jus réactionnels, on reprend le résidu dans 250 ml d'éthanol absolu, on obtient un précipité, on essore, on lave à l'éthanol absolu, on obtient un deuxième jet de 32 g, soit un rendement global de 32 %.

Le spectre IR confirme la structure.

### b) Préparation de l'acide N-bis (β-acétoxyéthyl) oxamique

On ajoute 90 g (0,42 mole) du composé obtenu en (a) dans 1800 ml d'acide acétique saturé en acide chlorhydrique gazeux. On agite à température ambiante pendant 48 heures, on essore l'insoluble (chlorure de potassium), puis on évapore le filtrat. On obtient une huile qui cristallise, on reprend dans 400 ml d'éther isopropylique, on laisse cristalliser une nuit sous agitation. Après essorage, lavage à l'éther isopropylique et séchage, on obtient 75 g de produit, soit un rendement de 69 %.

Le spectre IR confirme la structure.

### c) Préparation du chlorure de l'acide N-bis (β-acétaxy éthyl) oxamigue

On dissout 95 g (0,29 mole) du composé obtenu en (b) dans 250 ml de chlorure de thionyle, on porte à 80°C pendant 2 heures. On évapore le chlorure de thionyle, on reprend deux fois par 100 ml de benzène qu'on élimine sous vide. On obtient un concentrat huileux qu'on utilise sans autre purification.

### d) Préparation de la 2,4,6-triiodo-3,5-bis [bis-acétoxy éthyl) carbamoyl7aniline

A 0,1 mole de chlorure de l'acide 5-amino-2,4,6- triodo isophtalique dissous dans 10 ml de DMAC, on ajoute à 20-25°C, 6,8 g de chlorhydrate de la N-bis (β-acétoxy éthyl)amine (obtenu à l'exemple 1(d)a) et 7,7 ml de triéthylamine. Après 16 h d'agitation à température ambiante le chlorhydrate de triéthylamine est essoré et la solution réactionnelle est versée sur de l'eau. La gomme obtenue est dissoute dans le dichloroéthane et la solution est lavée deux fois à l'eau, séchée et évaporée à sec. Rendement: 70 %.

CCM : Eluant 3 Rf : 0,8 et 0,9 (2 isomères)

### e) Préparation du 5-[N-bis (β-acétoxyéthyl) carbamoyl- carbonyl-amino-2,4,6-triiodo-N,N'-bis /bis (β-acétoxy éthyl)] isophtalamide

On dissout 91,1 g (0,1 mole) de 2,4,6-triiodo-3,5- bis [bis (acétoxyéthyl) carbamoyl] aniline dans 150 ml de diméthylacétamide. On ajoute 84 g (0,3 mole) de chlorure d'acide obtenu en (c).

On laisse agiter 24 heures à température ambiante. On verse dans 750 ml d'eau + glace, on obtient un précipité gommeux. On décante les jus surnageants et le résidu gommeux est repris dans 250 ml d'éther diméthylique de l'éthylène glycol. On laisse cristalliser pendant 24 heures. Après essorage et séchage, on obtient 85 g de produit, soit un rendement de 73,5 %.
CCM : Eluant 9

Rf : 0,7.

### f) Saponification

Les 85 g de produit obtenu précédemment sont dissous dans 260 ml de soude 2N..Après 4 heures d'agitation à température ambiante, on ajoute 1 litre d'eau et 400 ml de résine cationique Amberlite IRN77. Après filtration, les eaux mères sont traitées deux fois au noir (2 %) 3SA pendant 2 heures à température ambiante. Après filtration, le filtrat est traité par 600 ml de résine anionique Amberlite IRN78. La suspension est filtrée et les jus évaporés. Le produit amorphe obtenu est lavé à l'éthanol. Après essorage et séchage, on a un produit blanc et un rendement de 47 %.
CCM : Eluant 7

Rf : 0,.28 et 0,32.

Dosage d'iode : 98,5 %.

### EXEMPLES 10 à 12

En opérant comme à l'exemple 9, on a obtenu les composés indiqués dans le tableau I.

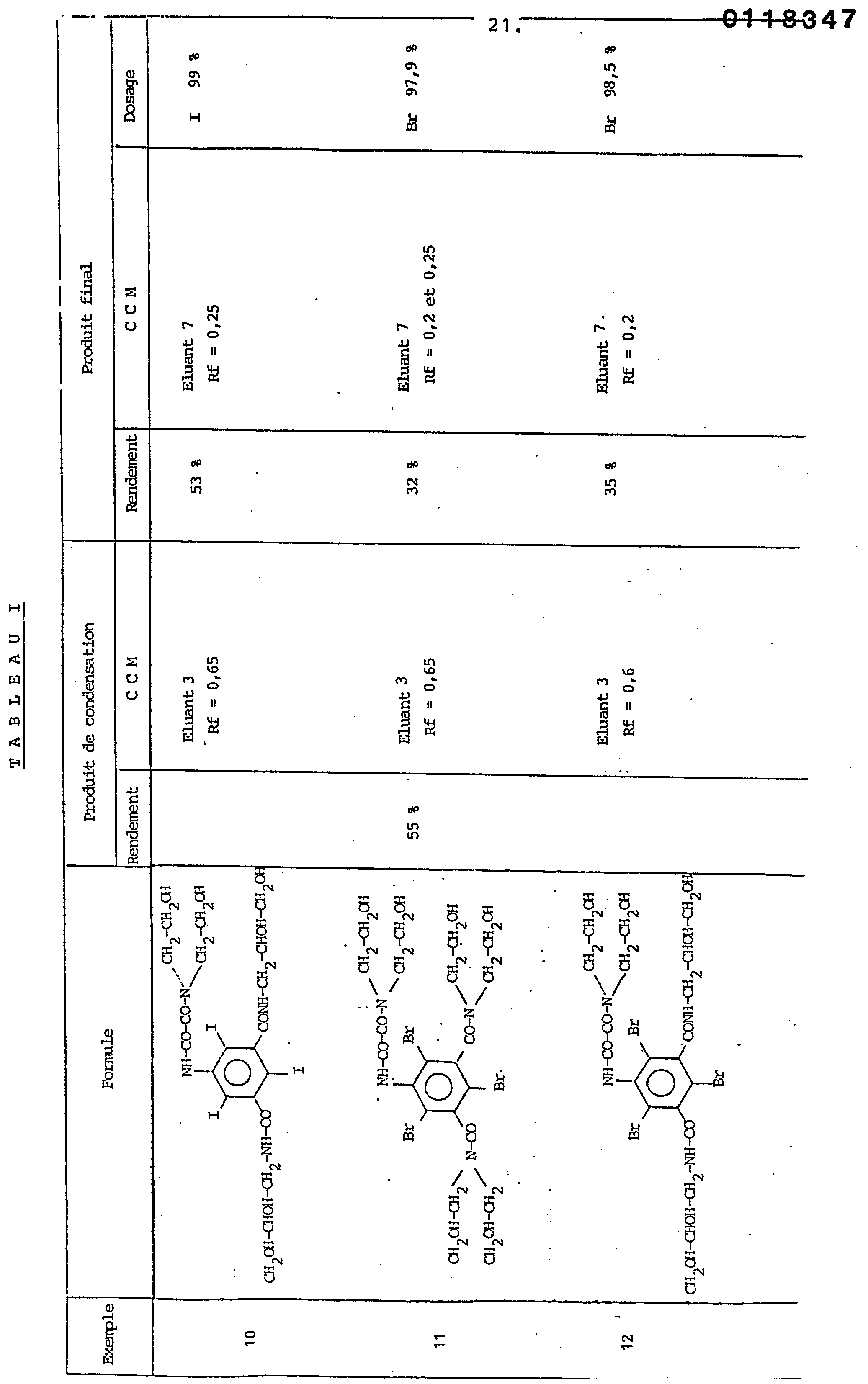

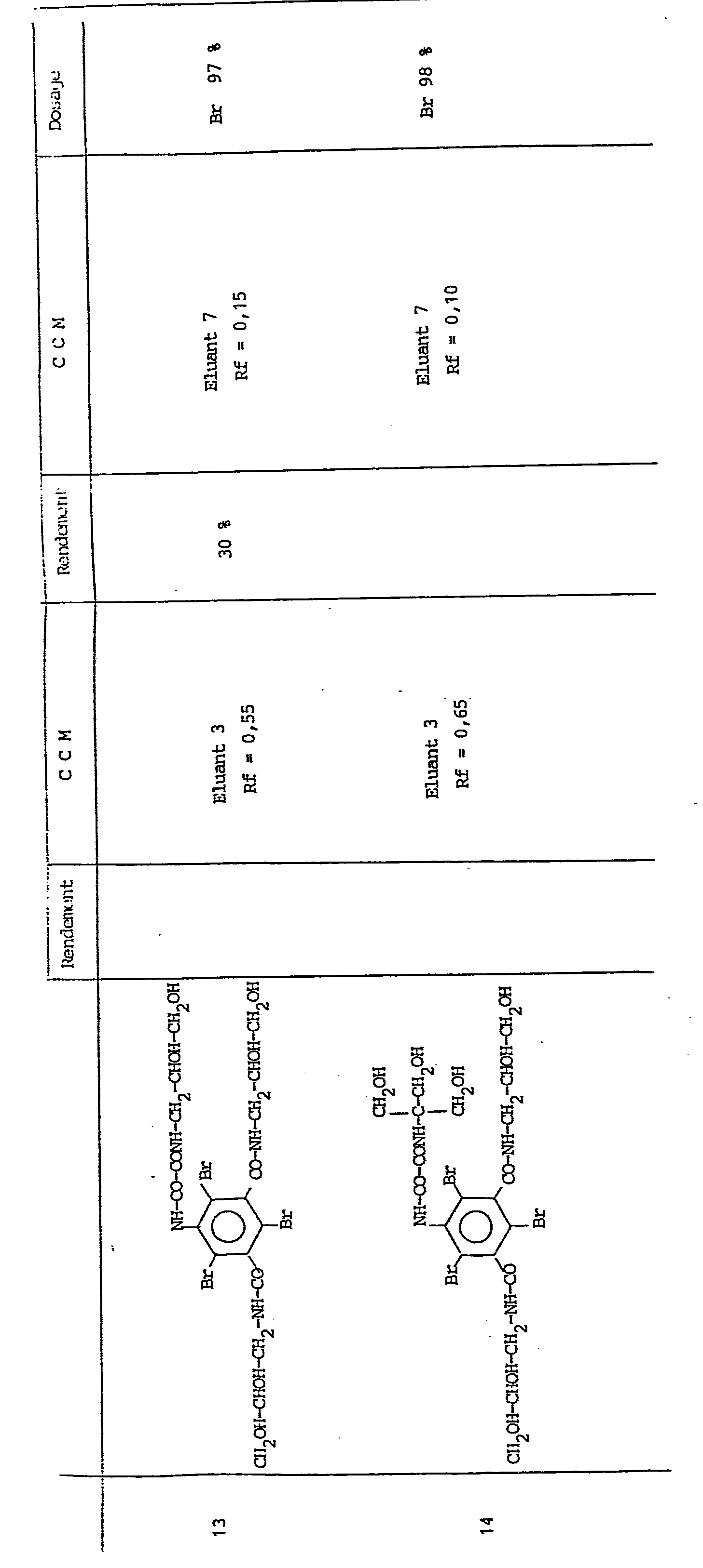

### EXEMPLE 15

### Préparation du 5-[N-bis (β-hydroxyéthyl) carbamoylméthyl-N-méthoxyacétyl-amino]-2,4,6-tribromo-N,N'-bis /bis (β-hydroxyéthyl)] isophtalamide

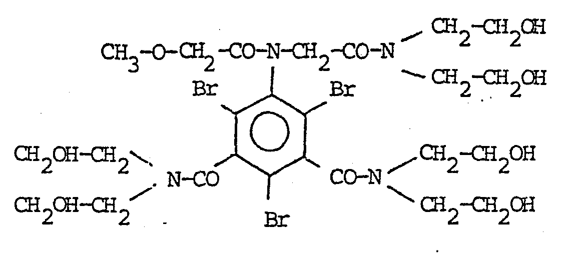

### a) Préparation du chlorure de l'acide-5-(N-méthoxyacétyl- amino)-2,4,6-tribromo isophtalique

On ajoute goutte à goutte et sous agitation à une température inférieure à 10°C, 22,7 g (0,05 mole) de chlorure d'acide 5-amino-2,4,6-tribromo isophtalique en suspension dans 75 ml de CHCl3 en présence de 8 ml (0,055 mole) de triéthylamine, sur une solution chloroformique contenant 0,25 mole de chlorure d'acide méthoxy acétique: celui-ci est fabriqué in situ dans CHCl₃ à partir de 18,7 ml (0,25 mole) d'acide méthoxyacétique et 18 ml (0,25 mole) de chlorure de thionyle par agitation 1 heure à température inférieure à 10°C.

Le mélange réactionnel est chauffé à reflux du chloroforme une journée jusqu'à disparition du produit de départ'. Après lavage à l'eau bicarbonatée, séchage et évaporation de la phase chloroformique, on obtient 19,6 g d'un solide blanc, soit un rendement de 74,5 %, avec un point de fusion net F : 145°C.
CCM : Eluant 8

Rf. 0,87.

### b) Préparation du 5-méthoxyacétyl-amino-2,4,6-tribromo- _{N},_{N}'-bis /bis (β-hydroxyéthyl)] isophtalamide

42 g (0,08 mole) du chlorure d'acide obtenu en (a) sont dissous dans 40 ml d'acétone. La diéthanolamine en solution dans l'eau et 0,24 mole de bicarbonate de potassium sont ajoutés progressivement en maintenant la température du milieu réactionnel autour de 20°C. Après plusieurs jours à température ambiante, le produit cristallisé est filtré, lavé plusieurs fois à l'éther diéthylique, puis séché à l'étuve.

### c) Préparation du 5-(méthoxyacétyl-amino)-2,4,6-tribromo N,N'-bis Ibis (β-acétoxyéthyl)]isophtalamide

Le composé obtenu en (b) est dissous dans l'éther diméthylique de l'éthylène glycol. Après l'addition de chlorure d'acétyle, la solution est chauffée à 80°C pendant une heure. La solution est ensuite refroidie à température ambiante. Après plusieurs jours sous agitation, le produit-cristallisé est filtré, lavé à l'éther diéthylique, puis séché sous vide.

### d) Préparation du 5-[N-bis (β-hydroxyéthyl) carbamoyl méthyl-N-méthoxyacétyl]-2,4,6-tribromo-N,N'-bis Ibis (β-hyrdroxyéthyl)] isophtalamide

Le composé obtenue en (c) est ajouté à une suspension de NaH dans le diméthyl acétamide anhydre. Après une heure de réaction (dégagement d'hydrogène), on ajoute du N-di(acétoxyéthyl)-α-bromoacétamide dilué dans du DMAC.

Le mélange réactionnel est laissé une nuit à température ambiante, puis évaporé à sec.

L'huile résultante est saponifiée par dissolution dans l'éthanol additionné de soude 2N. La solution est ensuite traitée successivement par une résine cationique (Amberlite IRN77) et par une résine anionique (Amberlite IRN78). Après filtration de la résine, les jus sont évaporés à sec.

Les spectres IR et ¹HRMN confirment la structure.

### EXEMPLE 16

### Préparation du 5-[N-bis (S-hydroxyéthyl) carbamoylméthyl-N-méthoxyacétyl-amino]-2,4,6-triiodo-N,N'-bis /bis (β-hydroxyéthyl)] isophtalamide

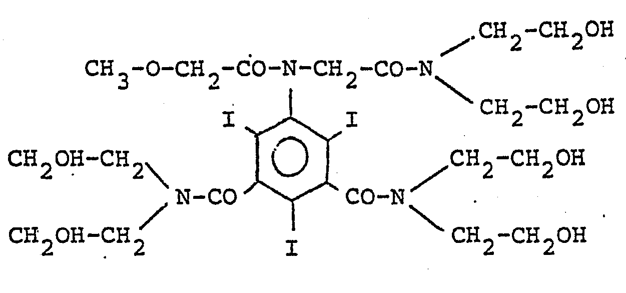

Il est obtenu comme décrit à l'exemple 15.

### EXEMPLE 17

### Préparation du 5-/N-tris (hydroxyméthyl) méthyl carbamoyl méthyl-N-acétyl-amino]-2,4,6-tribromo-N,N'-bis (2,3-_{d}i-hydroxy propyl) isophtalamide

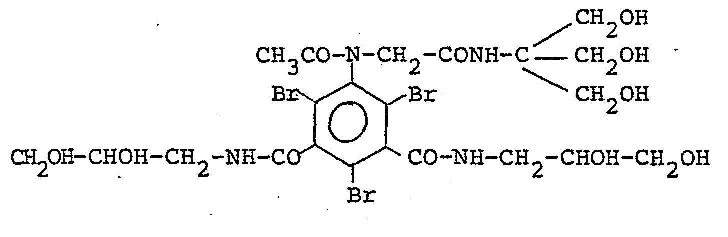

### a) Préparation du chlorure de l'acide 5-acétamido -2,4,6-tribromo. isophtalique

25,6 ml (0,36 mole) de chlorure d'acétyle sont ajoutés goutte à goutte à une solution contenant 110 g (0,24 mole) de chlorure d'acide 5-amino-2,4,6-tribromo isophtalique dans 120 ml de diméthylacétamide à 0°C.

La solution est chauffée 2 heures à 40°C, puis agitée à température ambiante. Le produit blanc qui cristallise peu à peu est essoré après 16 h, lavé à l'eau additionnée de quelques gouttes de soude jusqu'à pH 7, puis lavé à l'éther isopropylique. Rendt. 39,5 %.

Un deuxième jet est obtenu par précipitation des eaux mères à l'eau glacée ce qui donne un rendement global de 83 %. C_{CM :} le produit condensé à la propylamine migre à un Rf de 0,6 dans l'éluant Benzène/méthyléthylcétone/acide formique (50/25/20)

Structure confirmée par spectre IR et RMN.

### b) Préparation du 5-acétamido-2,4,6-tribromo-N,N'-bis-(2,3-dihydroxypropyl) isophtalamide

40 g (0,080 mole) du chlorure de l'acide 5-acétamido-2,4,6-tribromo isophtalique dissous dans 40 ml d'acétone sont ajoutés goutte à goutte dans une solution de 3-aminopropane-1,2-diol (0,24 mole) dans 40 ml d'eau en présence de 0,24 mole de bicarbonate de potassium. On agite 16 heures à température ambiante. On essore le produit qui a cristallisé et on le lave trois fois à l'éthanol afin d'éliminer l'excès d'aminopropanediol.

Puis, le produit est dissous au reflux dans le méthanol, les sels minéraux sont éliminés par filtration à chaud et le produit purifié recristallise dans le méthanol.
CCM: Eluant 7 - Rf = 0,35.
Eluant 2 - Rf = 0,3.

### c) Préparation du 5-/N-tris (hydroxyméthyl) méthyl carbamoyl méthyl-N-acétyl-amino]-2,4,6-tribromo-N,N'-bis (2,3-dihydroxy propyl) isophtalamide

Il est obtenu comme décrit à l'exemple 4 en utilisant le composé obtenu en (b) à la place du 5-acétamido-2,4,6-tribromo-N,N'-bis [bis(β-acétoxy éthyl)] isophtalamide.

CCM : Eluant 7 - Rf = 0,35
Eluant CACl₃/MeOH (60/40) Rf = 0,35.

### EXEMPLE 18

### Préparation du 5-[N-bis (β-hydroxyéthyl)-carbamoyl méthyl-N-acétyl amino]-2,4,6-tribromo-N,N'-bis (2ₜ3-dihydroxy propyl) isophtalamide

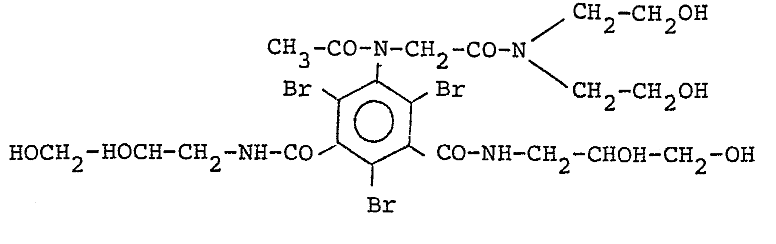

Il est obtenu comme décrit dans l'exemple 2 en utilisant le 5-acétamido-2,4,6-tribromo-N,N'-bis-(2,3-dihydroxy propyl) isophtalamide (exemple 17b) à la place du 5-acétamido-2,4,6-tribromo-N,N'-bis /bis (β-acétoxy éthyl)/ isophtalamide.
CCM : Eluant 7 - Rf = 0,32
CHCl₃/méthanol (60/40) Rf = 0,45.

### EXEMPLE 19

### Préparation du 5-[N-bis (β-hydroxyéthyl) carbamoyl méthyl-N-méthoxyacétyl-amino]-2,4,6-tribromo-N,N'-bis-(2,3-dihydroxy propyl) isophtalamide

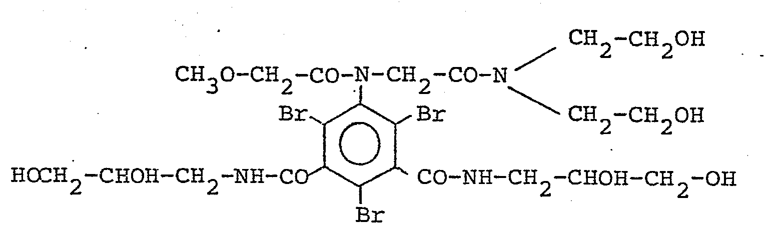

### a) Préparation du 5-(N-méthoxyacétyl-amino)-2,4,6-tribromo-N,N'-bis (2,3-dihydroxypropyl)- isophtalamide

42 g (0,08 mole) du chlorure de l'acide 5 méthoxy-acétyl amino-2, 4, 6 -tribromo isophtalique en solution dans 40 ml d'acétone sont ajoutés goutte à goutte à une solution de 0,24 mole de 3 aminopropane-1,2-diol dans 40 ml d'eau en présence de 0,24 mole de bicarbonate de potassium. Le mélange réactionnel est agité 16 heures à température ambiante. Le produit cristallisé est essoré, puis lavé trois fois à l'éthanol.

Après dissolution du produit au reflux du méthanol, suivie d'une filtration à chaud afin d'éliminer les sels minéraux, le produit est purifié par recristallisation dans lé méthanol.
CCM : Butanol/eau/CH₃COOH (50/25/11) Rf : 0,46
Benzène/MEC/HCOOH (60/25/10) Rf : 0,04

La structure est confirmé par spectrométrie IR et ¹HRMN.

### b) Préparation du 5-[N-bis (β-hydroxyéthyl) carbamoyl méthyl-N-méthoxyacétyl-amino ]- 2,4,6- tribromo -N,N' bis (2,3-dihydroxy-propyl) isophtalamide

Il est obtenu comme décrit dans l'exemple 15 en utilisant le composé obtenu en b.

## Revendications

1. Composés de formule 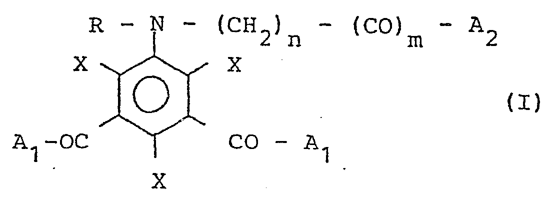 dans laquelle
X est un atome d'iode ou de brome,
R est un atome d'hydrogène, un groupe acétyle, méthoxyacétyle ou trifluoroacétyle,
m est un nombre entier de 0 à 2,
n est un nombre entier de 0 à-4, la somme m + n étant au moins égale à 2,
A₁ et A₂ représentent indépendamment l'un de l'autre un reste d'aminoalcool de formule 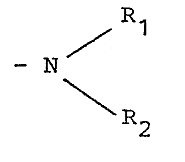
dans laquelle
R₁ représente un atome d'hydrogène, un groupe alcoyle en C₁ à C₆ ou un groupe alcoyle en C₁ à C₆ mono-ou polyhydroxylé, et
R₂ représente un groupe alcoyle en C₁ à C₆ mono-ou polyhydroxylé.

2. Composés selon la revendication 1 de formule. 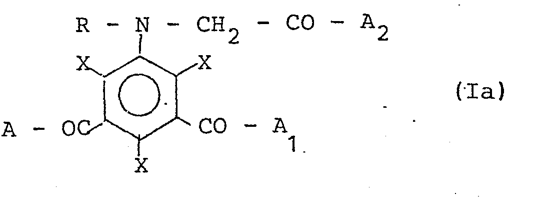 dans laquelle X, R, A₁ et A₂ ont la signification donnée à la revendication 1.

3. Composés selon la revendication 1, de formule 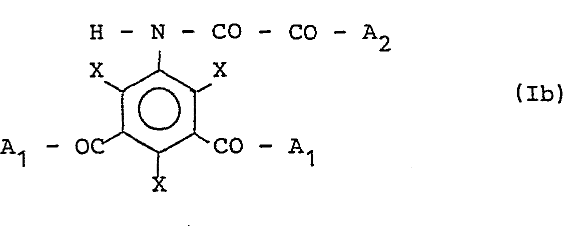 dans laquelle X, A₁ et A₂ ont la signification donnée à la revendication 1.

4. Composés selon l'une quelconque des revendications 1 à 3, caractérisés en ce que les restes d'amino- alcool A₁ et A₂ comprennent au moins deux groupes hydroxy.

5. Le 5-/N-bis (β-hydroxyéthyl) carbamoylméthyl-N-acétyl-amino]-2,4,6-triiodo-N,N'-bis [bis (β-hydroxyéthyl)] isophtalamide.

6. Le 5-[N-bis(β-hydroxyéthyl) carbamoylméthyl-N-acétyl-amina]-2,4,6-tribromo-N,N'-bis /bis (β-hydroxyéthyl)]isophtalamide.

7. Produit opacifiant comprenant un composé selon l'une quelconque des revendications 1 à 6.

8. Produit opacifiant selon la revendication 7, caractérisé en ce qu'il comprend un mélange d'un composé bromobenzénique de formule I et d'un composé iodobenzénique.

9. Produit opacifiant selon la revendication 8, caractérisé en ce qu'il comprend un mélange d'un composé bromobenzénique de formule I avec un composé iodobenzénique de formule I.

10. Produit opacifiant selon l'une quelconque des revendications 8 ou 9 sous forme de solution aqueuse.

1. Procédé de préparation de composés de formule 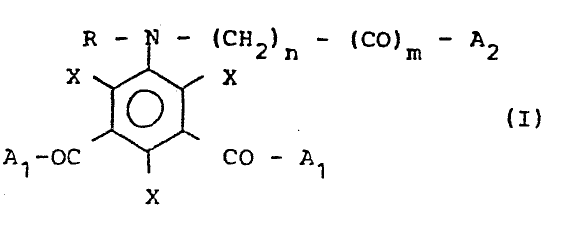 dans laquelle
X est un atome d'iode ou de brome,
R est un atome d'hydrogène, un groupe acétyle, méthoxyacétyle ou trifluoroacétyle,
m est un nombre entier de 0 à 2,
n est un nombre entier de 0 à 4, la somme m + n étant au moins égale à 2,
A₁ et A₂ représentent indépendamment l'un de l'autre un reste d'aminoalcool de formule 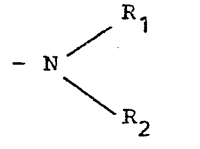
dans laquelle
R₁ représente un atome d'hydrogène, un groupe alcoyle en C₁ à C₆ ou un groupe alcoyle en C₁ à C₆ mono-ou polyhydroxylé, et
R₂ représente un groupe alcoyle en C₁ à C₆ mono-ou polyhydroxylé,
caractérisé en ce que :
1 - on effectue une condensation d'un composé de formule 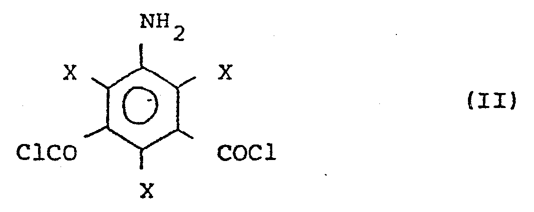 dans laquelle X a la signification donnée ci-dessus, avec une amine de formule 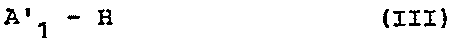 dans laquelle A'₁ désigne un groupe A dont les groupes hydroxy sont protégés conduisant à une amine de formule 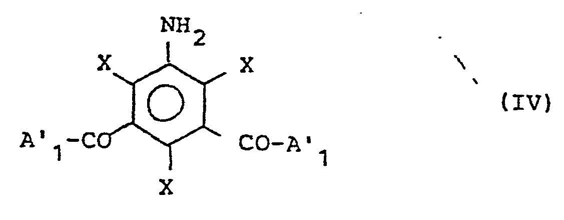 et, éventuellement, on effectue une acylation de l'amine ainsi obtenue par un chlorure d'acide de formule R'Cl (V) dans laquelle R' représente un groupe R de type acyle, obtenant ainsi un composé de formule 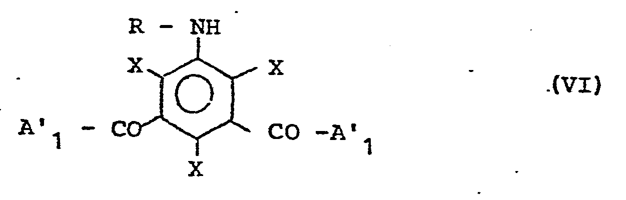 ou bien
2 - on effectue une condensation d'un composé de formule (II) avec une amine de formule A₁-H (XI) conduisant à une amine 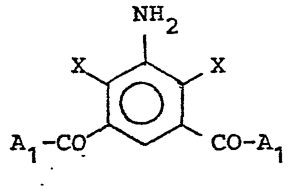 puis on effectue une acylation de l'amine ainsi obtenue avec un chlorure d'acide de formule (V) conduisant à un composé de formule VI ou bien
3 - on effectue une acylation de l'amine de formule (II) avec un chlorure d'acide de formule (V), conduisant à un dichlorure d'acide de formule 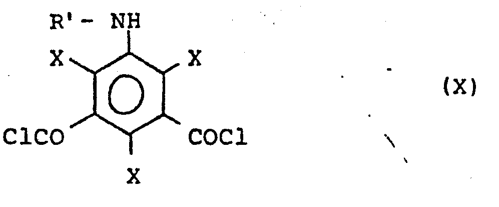 puis on effectue une condensation du dichlorure d'acide ainsi obtenu avec une amine de formule (III) conduisant à un composé de formule (VI) ou avec une amine non protégée de formule (XI) et l'on protège ensuite les groupes hydroxy,
4 - on fait réagir le composé de formule (VI) ainsi obtenu avec soit un composé de formule 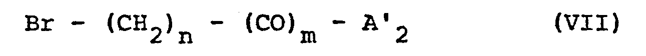 dans laquelle A'₂ est un groupe A₂ dont les groupes hydroxy sont protégés et n > 0, soit un composé de formule 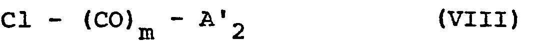 dans laquelle AI2 est un groupe A₂ dont les groupes hydroxy sont protégés
conduisant à un composé de formule 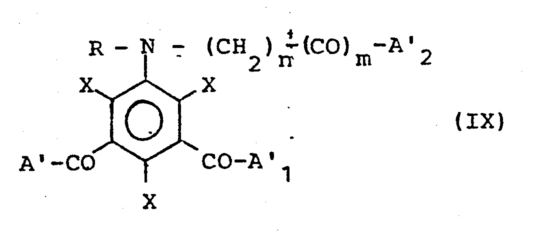
et
5 - on élimine les groupes protecteurs.

2. Procédé selon la revendication 1, caractérisé en ce que, comme composé de formule VII on utilise un composé de formule : 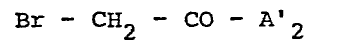 et l'on obtient un composé de formule :

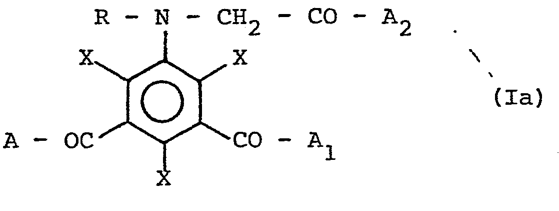

3. Procédé selon la revendication 1, caractérisé en ce que, comme composé de formule (VIII) on utilise un composé de formule : 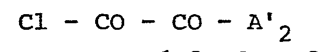 et l'on obtient un composé de formule :

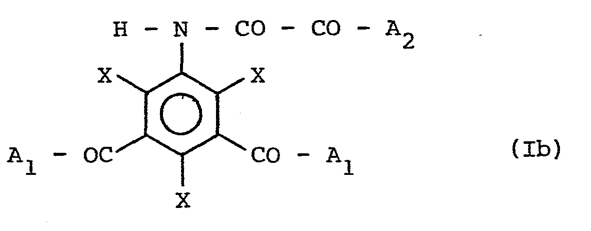

4. Procédé de préparation de produits opacifiants, caractérisé en ce que l'on met un composé de formule I, Ia ou Ib, telles que définies aux revendications 1 à 3, sous une forme convenant pour une administration chez l'homme.

5. Procédé de préparation de produits opacifiants selon la revendication 4, caractérisé en ce que l'on met un composé bromobenzénique de formule I, Ia ou _{I}b, telles que définies aux revendications 1 à 3, en mélange avec un composé iodobenzénique sous une forme convenant pour une administration chez l'homme.

6. Procédé de préparation de produits opacifiants selon la revendication 4 ou la revendication 5, caractérisé en ce que l'on met un composé bromobenzénique de formule I, Ia ou Ib, telles que définies aux revendications 1 à 3, en mélange avec un composé iodobenzénique de formule I, la ou Ib, telles que définies aux revendications 1 à 3, sous une forme convenant pour une administration chez l'homme.
